(19) Europäisches Patentamt
European Patent Office
Office européen des brevets

(11) **EP 3 724 174 B1**

(12) **EUROPÄISCHE PATENTSCHRIFT**

(45) Veröffentlichungstag und Bekanntmachung des Hinweises auf die Patenterteilung:
**09.02.2022   Patentblatt 2022/06**

(21) Anmeldenummer: **18829226.2**

(22) Anmeldetag: **07.12.2018**

(51) Internationale Patentklassifikation (IPC):
**C07D 317/38** (2006.01)

(52) Gemeinsame Patentklassifikation (CPC):
**C07D 317/38**

(86) Internationale Anmeldenummer:
**PCT/EP2018/084013**

(87) Internationale Veröffentlichungsnummer:
**WO 2019/115399 (20.06.2019 Gazette 2019/25)**

(54) **VERFAHREN ZUR HERSTELLUNG VON GLYCERINCARBONATMETHACRYLAT**

METHOD FOR THE PRODUCTION OF GLYCERINE CARBONATE METHACRYLATE

PROCÉDÉ DE PRODUCTION DU MÉTHACRYLATE DE CARBONATE DE GLYCÉROL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.12.2017   EP 17207791**

(43) Veröffentlichungstag der Anmeldung:
**21.10.2020   Patentblatt 2020/43**

(73) Patentinhaber: **BASF SE**
**67056 Ludwigshafen am Rhein (DE)**

(72) Erfinder:
• **MISSKE, Andrea**
**67056 Ludwigshafen (DE)**
• **FLECKENSTEIN, Christoph**
**67056 Ludwigshafen (DE)**
• **KLUEGLEIN, Matthias**
**67056 Ludwigshafen (DE)**

(74) Vertreter: **BASF IP Association**
**BASF SE**
**GBI-C006**
**67056 Ludwigshafen (DE)**

(56) Entgegenhaltungen:
**EP-A2- 0 212 409**

• **THOMAS WERNER ET AL:** "Hydroxyl-Functionalized Imidazoles: Highly Active Additives for the Potassium Iodide-Catalyzed Synthesis of 1,3-Dioxolan-2-one Derivatives from Epoxides and Carbon Dioxide", CHEMCATCHEM, Bd. 6, Nr. 12, 10. Oktober 2014 (2014-10-10), Seiten 3493-3500, XP055450067, DE ISSN: 1867-3880, DOI: 10.1002/cctc.201402572
• **THOMAS WERNER ET AL:** "Synthesis of cyclic carbonates from epoxides and CO2 catalyzed by potassium iodide and amino alcohols", JOURNAL OF CO2 UTILIZATION, Bd. 7, 1. September 2014 (2014-09-01), Seiten 39-45, XP055450078, ISSN: 2212-9820, DOI: 10.1016/j.jcou.2014.04.002
• **SONG JINLIANG ET AL:** "Highly efficient synthesis of cyclic carbonates from CO2and epoxides catalyzed by KI/lecithin", CATALYSIS TODAY, Bd. 183, Nr. 1, 23. September 2011 (2011-09-23), Seiten 130-135, XP028902807, ISSN: 0920-5861, DOI: 10.1016/J.CATTOD.2011.08.042

**Beschreibung**

Die vorliegende Erfindung betrifft ein Verfahren zur Herstellung von Glycerincarbonatmethacrylat (GCMA). Bei dem erfindungsgemäßen Verfahren wird Glycidylmethacrylat (GMA) in Gegenwart von Kaliumiodid als Katalysator mit Kohlenstoffdioxid umgesetzt.

Glycerincarbonatmethacrylat wird beispielsweise zur Herstellung von Copolymeren verwendet. Neben Glycerincarbonatmethacrylat enthalten diese Copolymere bevorzugt auch Acrylate, Methacrylate und/oder Vinylmonomere in einpolymerisierter Form.

Über die Reaktivität der Carbonatgruppe des einpolymerisierten Glycerincarbonatmethacrylats ist es möglich, Glycerincarbonatmethacrylat enthaltende Copolymere zu modifizieren und damit in ihren Eigenschaften gezielt zu verändern.

So werden Copolymere, die Glycerincarbonatmethacrylat als Comonomer in einpolymerisierter Form enthalten, beispielsweise als Vernetzer eingesetzt. Die Carbonatgruppe des einpolymerisierten Glycerincarbonatmethacrylat ermöglicht beispielsweise Vernetzungsreaktionen mit Polymeren, die freie Aminogruppen, Hydrazidgruppen, Hydrazongruppen, Carbonsäuregruppen, Anhydridgruppen und/oder Hydroxygruppen aufweisen.

Verfahren zur Herstellung von Glycerincarbonatmethacrylat durch Umsetzung von Glycidylmethacrylat mit Kohlenstoffdioxid in Gegenwart eines Katalysators sind dem Fachmann aus dem Stand der Technik bekannt. Als Katalysatoren werden dabei meist Iodide und/oder Aminverbindungen eingesetzt. In Abhängigkeit der eingesetzten Katalysatoren kann die Umsetzung bei Atmosphärendruck oder erhöhtem Druck erfolgen.

Aufgabe war es, ein verbessertes Verfahren zur Herstellung von Glycerincarbonatmethacrylat zur Verfügung zu stellen. Durch das verbesserte Verfahren sollte es möglich sein, Glycerincarbonatmethacrylat unter Verwendung eines einfach zugänglichen Katalysators, wie Kaliumiodid, bei niedrigen Drücken in hoher Selektivität herzustellen. Zudem sollte eine schnellere Umsetzung des eingesetzten GMA zu GCMA möglich sein. Niedrige Drücke stellen in diesem Zusammenhang Drücke von 0,5 bis 5, bevorzugt 0,8 bis 1,5 und besonders bevorzugt 0,8 bis 1,2 bar dar. Eine hohe Selektivität stellt eine Selektivität über 95%, bevorzugt über 97 % ganz besonders bevorzugt über 97,5% dar.

Die Aufgabe wird gelöst durch ein Verfahren zur Herstellung von Glycerincarbonatmethacrylat, wobei Glycidylmethacrylat in Gegenwart von einem Katalysator und eines Lösungsmittels mit Kohlenstoffdioxid umgesetzt wird, das dadurch gekennzeichnet ist, dass es sich bei dem Katalysator um Kaliumiodid handelt, es sich bei dem Lösungsmittel um Acetonitril, ein oder mehrere Mono-Alkohole, oder um eine beliebige Mischung aus Acetonitril und einem oder mehreren Mono-Alkoholen handelt, und die Umsetzung von Glycidylmethacrylat mit Kohlenstoffdioxid bei einem Druck von 0,5 bis 5 bar erfolgt.

Definitionen:

Die Abkürzung GMA steht für Glycidylmethacrylat (2,3-Epoxypropylmethacrylat).

Die Abkürzung GCMA steht für Glycerincarbonatmethacrylat (2-Oxo-1,3-dioxolan-4-yl)methylmethacrylat.

Die Selektivität berechnet sich aus der nachfolgenden Formel:

$$Selektivität(\%) = \frac{GCMA(Fl.\%) \times 100}{GCMA(Fl.\%) + \sum Nebenprodukte(Fl.\%)}$$

Das erfindungsgemäße Verfahren dient der Herstellung von GCMA durch Umsetzung von GMA mit Kohlenstoffdioxid in Gegenwart von Kaliumiodid als Katalysator und eines Lösungsmittels. Es ist bevorzugt, dass Kohlenstoffdioxid einer Reaktionsmischung enthaltend den Katalysator, das Lösungsmittel, noch nicht umgesetztes GMA und optional bereits gebildetes GCMA zugeführt wird.

Das erfindungsgemäße Verfahren kann kontinuierlich oder diskontinuierlich ausgeführt werden, wobei eine diskontinuierliche Verfahrensausführung bevorzugt ist. Im Falle einer diskontinuierlichen Verfahrensausführung ist es vorteilhaft, GMA, den Katalysator und das Lösungsmittel vorzulegen. Der vorgelegten Reaktionsmischung wird dann Kohlenstoffdioxid zugeführt.

Als Reaktoren für das erfindungsgemäße Verfahren eigenen sich alle Reaktortypen, die dem Fachmann für kontinuierliche oder diskontinuierliche Verfahren bekannt sind. Wird das erfindungsgemäße Verfahren diskontinuierlich ausgeführt, eignen sich insbesondere Rührkesselreaktoren. Mehrere Reaktoren, bevorzugt Rührkesselreaktoren bei einer diskontinuierlichen Verfahrensführung, können in Reihe und/oder parallelgeschaltet sein.

Die Umsetzung von GMA mit Kohlenstoffdioxid erfolgt bei einem Druck von 0,5 bis 5 bar. Es ist bevorzugt, dass die Umsetzung bei einem Druck von 0,8 bis 1,5 bar erfolgt. Es ist weiter bevorzugt, dass die Umsetzung bei einem Druck von 0,8 bis 1,2 bar erfolgt.

Die Umsetzung von GMA mit Kohlenstoffdioxid erfolgt bevorzugt bei einer Temperatur von 50 bis 100 °C und besonders bevorzugt bei einer Temperatur von 75 bis 100 °C.

Es ist demnach bevorzugt, dass die Umsetzung von GMA mit Kohlenstoffdioxid bei einem Druck von 0,8 bis 1,5 bar und einer Temperatur von 50 bis 100 °C und insbesondere bei einem Druck von 0,8 bis 1,5 bar und einer Temperatur von 75 bis 100°C erfolgt.

In dem erfindungsgemäßen Verfahren wird bevorzugt möglichst reines Glycidylmethacrylat eingesetzt. Möglichst reines GMA weist eine Reinheit von 95 bis 100 Gewichtsprozent auf. Es ist weiter bevorzugt, dass das in dem erfindungsgemäßen Verfahren eingesetzte GMA eine Reinheit von 97 bis 100 Gewichtsprozent und besonders bevorzugt eine Reinheit von 99 bis 100 Gewichtsprozent aufweist. Die Gewichtsprozentangaben beziehen sich auf die Gesamtmenge des eingesetzten GMA.

Das in dem erfindungsgemäßen Verfahren eingesetzte GMA kann bereits einen oder mehrere Polymerisationsstabilisatoren enthalten. Enthält das eingesetzt GMA bereits einen oder mehrere Polymerisationsstabilisatoren sind diese in wirksamen Konzentrationen enthalten. So kann das eingesetzte GMA beispielsweise 20 bis 1000 ppm, bevorzugt 50 bis 300 ppm und besonders 80 bis 120 ppm Polymerisationsstabilisator enthalten. Die Angaben in ppm beziehen sich auf die Gesamtgewichtsmenge an Polymerisationsstabilisator, die in dem eingesetzten GMA enthalten ist.

Geeignete Polymerisationsstabilisatoren sind dem Fachmann bekannt oder erschließen sich ihm aus seinem allgemeinen Fachwissen. Geeignete Polymerisationsstabilisatoren sind beispielsweise Kupfer(meth)acrylate, Kupferdithiocarbamate, Phenothiazine, phenolische Verbindungen, N-Oxyle, Phenylendiamine, Nitrosoverbindungen, Harnstoffe oder Thioharnstoffe. Diese Polymerisationsstabilisatoren können einzeln oder als beliebige Mischung eingesetzt werden. Bevorzugte Stabilisatoren sind Phenothiazine, phenolische Verbindungen, N-Oxyle oder beliebige Mischungen dieser.

Phenothiazine können beispielsweise Phenothiazin, Bis-(a-methylbenzyl)phenothiazin, 3,7-Dioctylphenothiazin, Bis-(a-dimethylbenzyl)phenothiazin oder eine beliebige Mischung dieser sein.

Phenolische Verbindungen können beispielsweise Hydrochinon, Hydrochinonmonomethylether, wie para-Methoxyphenol (MEHQ), Pyrogallol, Catechol, Resorcin, Phenol, Kresol, 2,4-Dimethyl-6-tert-butylphenol, 2,6-Di-tert-butyl-para-kresol oder eine beliebige Mischung dieser sein. Bevorzugte phenolische Verbindungen sind para-Methoxyphenol (MEHQ), 2,4-Dimethyl-6-tert-butylphenol, 2,6-Di-tert-butyl-para-kresol oder eine beliebige Mischung dieser.

N-Oxyle können beispielsweise Di-tert-butylnitroxid, 2,2,6,6-Tetramethyl-4-hydroxypiperidyl-1-oxyl, 2,2,6,6-Tetramethylpiperidyl-1-oxyl, 2,2,6,6-Tetramethylpiperidinoxyl, 4-Hydroxy-2,2,6,6-tetramethylpiperidinoxyl, 4,4',4"-Tris-1-(2,2,6,6-tetramethylpiperidinoxyl)phosphite oder eine beliebige Mischung dieser sein.

In dem erfindungsgemäßen Verfahren wird Kaliumiodid als Katalysator eingesetzt. Es ist bevorzugt, dass das Molverhältnis zwischen der Gesamtmenge des eingesetzten Katalysators und der Gesamtmenge des eingesetzten GMA 0,005 : 1 bis 0,5 : 1 beträgt. Es ist weiter bevorzugt, dass das Molverhältnis 0,01 : 1 bis 0,3 : 1 beträgt.

Bei dem Lösungsmittel handelt es sich um Acetonitril, ein oder mehrere Mono-Alkohole oder eine beliebige Mischung aus Acetonitril und einem oder mehreren Mono-Alkoholen.

Es ist bevorzugt, dass die Gesamtmenge des eingesetzten Lösungsmittel 0,1 bis 50 Gewichtsprozent bezogen auf die Gesamtmenge des eingesetzten GMA beträgt. Es ist weiter bevorzugt, dass die Gesamtmenge des eingesetzten Lösungsmittel 0,5 bis 40 Gewichtsprozent bezogen auf die Gesamtmenge des eingesetzten GMA beträgt.

Bei einem Mono-Alkohol handelt es sich um einen einwertigen Alkyl-Alkohol. Es ist bevorzugt, dass es sich bei einem Mono-Alkohol um Ethanol, n-Propanol, iso-Propanol, n-Butanol, sek-Butanol, iso-Butanol oder tert-Butanol handelt. Es ist besonders bevorzugt, dass es sich bei einem Mono-Alkohol um tert-Butanol handelt.

Es ist besonders bevorzugt, dass Acetonitril in einer Menge von 0,5 bis 40 Gewichtsprozent bezogen auf die Gesamtmenge des eingesetzten GMA als Lösungsmittel eingesetzt wird. Insbesondere ist es bevorzugt, dass Acetonitril in einer Menge von 2 bis 40 Gewichtsprozent bezogen auf die Gesamtmenge des eingesetzten GMA als Lösungsmittel eingesetzt wird.

Kohlenstoffdioxid wird dem erfindungsgemäßen Verfahren kontinuierlich oder diskontinuierlich zugeführt. Es ist bevorzugt, dass Kohlenstoffdioxid dem erfindungsgemäßen Verfahren kontinuierlich zugeführt wird. Eine kontinuierliche Zuführung von Kohlenstoffdioxid ist insbesondere bevorzugt, wenn das erfindungsgemäße Verfahren diskontinuierlich ausgeführt wird.

Das Molverhältnis zwischen der Gesamtmenge des eingesetzten Kohlenstoffdioxids und der Gesamtmenge des eingesetzten GMA beträgt bevorzugt 1 : 1 bis 100 : 1. Es ist weiter bevorzugt, dass das Molverhältnis 1 : 1 bis 10 : 1 beträgt.

Die Zuführgeschwindigkeit von Kohlenstoffdioxid kann über weite Bereiche variieren. Generell ist es von Vorteil, die Zuführgeschwindigkeit von Kohlenstoffdioxid derart anzupassen, dass der Anteil an Kohlenstoffdioxid in dem aus der Reaktion abgeführten Gasstrom möglichst gering ist. Die Zuführgeschwindigkeit von Kohlenstoffdioxid kann über den Reaktionsverlauf variieren.

Die Zuführgeschwindigkeit von Kohlenstoffdioxid beträgt bevorzugt 1 bis 100 $L(CO_2)/(kg(GMA_{eingesetzte\ Gesamtmenge})h)$, weiter bevorzugt 10 bis 60 $L(CO2)/(kg(GMA_{eingesetzte\ Gesamtmenge})h)$, besonders bevorzugt 10 bis 30 L(CO2)/(kg(GMA $_{eingesetzte\ Gesamtmenge}$)h).

Kohlenstoffdioxid wird dem erfindungsgemäßen Verfahren bevorzugt in Form eines kohlenstoffdioxidhaltigen Gases zugeführt. Das kohlenstoffdioxidhaltige Gas wird dabei in die Reaktionsmischung, enthaltend den Katalysator, das Lösungsmittel, noch nicht umgesetztes GMA und optional bereits gebildetes GCMA und/oder in den Gasraum oberhalb der Reaktionsmischung eingeleitet. Es ist bevorzugt, dass das kohlenstoffdioxidhaltige Gas kontinuierlich eingeleitet wird.

Die Zuführgeschwindigkeit des kohlenstoffdioxidhaltigen Gases kann über weite Bereiche variieren. Generell ist es von Vorteil die Zuführgeschwindigkeit des kohlenstoffdioxidhaltigen Gases derart anzupassen, dass der Anteil an Kohlenstoffdioxid in dem aus der Reaktion abgeführten Gasstrom möglichst gering ist. Die Zuführgeschwindigkeit des kohlenstoffdioxidhaltigen Gases kann über den Reaktionsverlauf variieren.

Die Zuführgeschwindigkeit des kohlenstoffdioxidhaltigen Gases beträgt bevorzugt 1 bis 100 L($CO_2$)/(kg(GMA $_{eingesetzte\ Gesamtmenge}$)h), weiter bevorzugt 10 bis 60 L(CO2)/(kg(GMA $_{eingesetzte\ Ge\text{-}samtmenge}$)h), besonders bevorzugt 10 bis 30 L(CO2)/(kg(GMA $_{eingesetzte\ Gesamtmenge}$)h).

Die Einleitung des kohlenstoffdioxidhaltigen Gases in die Reaktionsmischung kann beispielsweise über ein oder mehrere Tauchrohre oder ein oder mehrere Düsen erfolgen. Die Öffnungen der Tauchrohe oder Düsen liegen dabei unter der Oberfläche der Reaktionsmischung. Wird das kohlenstoffdioxidhaltige Gas in den Gasraum oberhalb der Reaktionsmischung eingeleitet, erfolgt dies beispielsweise über ein oder mehrere Zuleitungsrohre oder ein oder mehrere Düsen. Die Öffnungen der Zuleitungsrohre oder Düsen befinden sich dabei oberhalb der Oberfläche der Reaktionsmischung.

Im Falle einer diskontinuierlichen Verfahrensausführung kann das Einleiten des kohlenstoffdioxidhaltigen Gases vor dem Erwärmen der vorgelegten Reaktionsmischung auf eine Temperatur von 50 bis 100°C, während dem Erwärmen und/oder nach Erreichen der Zieltemperatur erfolgen. Es ist bevorzugt, dass die Einleitung des kohlenstoffdioxidhaltigen Gases über den gesamten Verfahrensverlauf erfolgt. Das heißt, dass das kohlenstoffdioxidhaltige Gas bereits vor dem Erwärmen oder während dem Erwärmen der Reaktionsmischung eingeleitet wird und die Einleitung des kohlenstoffdioxidhaltigen Gases erst beendet wird, wenn das eingesetzte GMA weitgehend zu GCMA umgesetzt ist. "Weitgehend" bedeutet in diesem Zusammenhang, dass 90 bis 100 Gewichtsprozent, bevorzugt 95 bis 100 Gewichtsprozent, besonders bevorzugt 98 bis 100 Gewichtsprozent des eingesetzten GMA zu GCMA umgesetzt sind.

Neben Kohlenstoffdioxid kann das kohlenstoffdioxidhaltige Gas auch ein oder mehrere von Kohlenstoffdioxid unterschiedliche Gase enthalten. Von Kohlenstoffdioxid unterschiedliche Gase sind beispielsweise Inertgase, wie Stickstoff, Argon oder Helium; oder Sauerstoff.

Der Kohlenstoffdioxidanteil in dem kohlenstoffdioxidhaltigen Gas beträgt bevorzugt 1 bis 100 Volumenprozent bezogen auf die Gesamtmenge des eingesetzten kohlenstoffdioxidhaltigen Gases. Es ist weiter bevorzugt, dass der Kohlenstoffdioxidanteil in dem kohlenstoffdioxidhaltigen Gas 50 bis 100 und besonders bevorzugt 90 bis 100 Volumenprozent beträgt.

Der Gesamtanteil an einem oder mehreren Gasen, die in dem kohlenstoffdioxidhaltigen Gas enthalten und von Kohlenstoffdioxid unterschiedlich sind beträgt bevorzugt 0 bis 99 Volumenprozent bezogen auf die Gesamtmenge des eingesetzten kohlenstoffdioxidhalten Gases. Es ist weiter bevorzugt, dass der Gesamtanteil an einem oder mehreren Gasen 0 bis 50 und besonders bevorzugt 0 bis 10 Volumenprozent beträgt.

Generell ist es möglich, wenn auch nicht bevorzugt, dass das kohlenstoffdioxidhaltige Gas neben einem oder mehreren Inertgasen und/oder Sauerstoff auch Spuren von anderen gasförmigen Stoffen wie Wasserstoff, Wasser, Methan und/oder Kohlenstoffmonoxid enthält. Die Summe aller im kohlenstoffdioxidhaltigen Gas enthaltenen Gase, gasförmige Stoffe eingeschlossen, addiert sich auf 100 Prozent.

Es ist bevorzugt, dass dem erfindungsgemäßen Verfahren ein sauerstoffhaltiges Gas zugeführt wird. Es ist bevorzugt, dass das sauerstoffhaltige Gas neben Sauerstoff ein oder mehrere von Sauerstoff unterschiedliche Gase enthält. Die Zuführung eines sauerstoffhaltigen Gases dient dazu, unerwünschte Polymerisation des eingesetzten GMA oder des hergestellten GCMA zu minimieren.

Das sauerstoffhaltige Gas kann dem Verfahren kontinuierlich oder diskontinuierlich zugeführt werden. Es ist bevorzugt, dass das sauerstoffhaltige Gas dem erfindungsgemäßen Verfahren kontinuierlich zugeführt wird. Eine kontinuierliche Zuführung eines sauerstoffhaltigen Gases ist besonders bevorzugt, wenn das erfindungsgemäße Verfahren diskontinuierlich ausgeführt wird.

Der Sauerstoffanteil in dem sauerstoffhaltigen Gas beträgt bevorzugt 1 bis 25 Volumenprozent bezogen auf die Gesamtmenge des eingesetzten sauerstoffhaltigen Gases. Es ist weiter bevorzugt, dass der Sauerstoffanteil in dem sauerstoffhaltigen Gas 5 bis 25 und besonders bevorzugt 10 bis 22 Volumenprozent beträgt.

Der Gesamtanteil an einem oder mehreren Gasen die in dem sauerstoffhaltigen Gas enthalten und von Sauerstoff unterschiedlich sind beträgt bevorzugt 75 bis 99 Volumenprozent bezogen auf die Gesamtmenge des eingesetzten sauerstoffhaltigen Gases. Es ist weiter bevorzugt, dass der Gesamtanteil an einem oder mehreren Gasen 75 bis 95 und besonders bevorzugt 78 bis 90 Volumenprozent beträgt. Von Sauerstoff unterschiedliche Gase sind beispielsweise Stickstoff, Argon, Helium oder Kohlenstoffdioxid. Der Anteil an Kohlenstoffdioxid in dem sauerstoffhaltigen Gas beträgt weniger als 1 Volumenprozent.

Bevorzugte sauerstoffhaltige Gase sind beispielsweise Luft, getrocknete Luft oder Magerluft.

Die Zuführgeschwindigkeit des sauerstoffhaltigen Gases kann über weite Bereiche variieren. Es ist bevorzugt, dass die Zuführgeschwindigkeit 0,1 bis 1, weiter bevorzugt 0,3 bis 0,8 und besonders bevorzugt 0,2 bis 0,4 $m^3/(m^3_{(Reaktionsmischung)}h)$ beträgt.

Wird das sauerstoffhaltige Gas dem erfindungsgemäßen Verfahren zugeführt, ist es bevorzugt, dass das sauerstoffhaltige Gas in die Reaktionsmischung und/oder in den Gasraum oberhalb der Reaktionsmischung eingeleitet wird. Die Einleitung in die Reaktionsmischung kann beispielsweise über ein oder mehrere Tauchrohre oder ein oder mehrere Düsen erfolgen. Die Öffnungen der Tauchrohre oder Düsen liegen dabei unter der Oberfläche der Reaktionsmischung.

Wird das sauerstoffhaltige Gas in den Gasraum oberhalb der Reaktionsmischung eingeleitet, erfolgt dies beispielsweise über ein oder mehrere Zuleitungsrohre oder ein oder mehrere Düsen. Die Öffnungen der Zuleitungsrohre oder Düsen befinden sich dabei oberhalb der Oberfläche der Reaktionsmischung.

Es ist demnach besonders bevorzugt, dass das sauerstoffhaltige Gas in die Reaktionsmischung und/oder in den Gasraum oberhalb der Reaktionsmischung kontinuierlich eingeleitet wird.

Das kohlenstoffdioxidhaltige Gas und das sauerstoffhaltige Gas können unabhängig voneinander in die Reaktionsmischung und/oder in den Gasraum oberhalb der Reaktionsmischung eingeleitet werden.

Im Falle einer diskontinuierlichen Verfahrensausführung kann das Einleiten des sauerstoffhaltigen Gases vor dem Erwärmen der vorgelegten Reaktionsmischung auf eine Temperatur von 50 bis 100°C, während dem Erwärmen und/oder nach Erreichen der Zieltemperatur erfolgen. Es ist bevorzugt, dass die Einleitung des sauerstoffhaltigen Gases über den gesamten Verfahrensverlauf erfolgt. Das heißt, dass das sauerstoffhaltige Gas bereits vor dem Erwärmen oder während dem Erwärmen der Reaktionsmischung eingeleitet wird und die Einleitung des sauerstoffhaltigen Gases erst beendet wird, wenn das eingesetzte GMA weitgehend zu GCMA umgesetzt ist. "Weitgehend" bedeutet in diesem Zusammenhang, dass 90 bis 100 Gewichtsprozent, bevorzugt 95 bis 100 Gewichtsprozent, besonders bevorzugt 98 bis 100 Gewichtsprozent des eingesetzten GMA zu GCMA umgesetzt sind.

Um unerwünschte Polymerisation des eingesetzten GMA und/oder des hergestellten GCMA im Rahmen des erfindungsgemäßen Verfahrens zu vermeiden, ist es bevorzugt, dass unabhängig davon ob das eingesetzte GMA einen oder mehrere Polymerisationsstabilisatoren enthält, die Umsetzung von GMA mit Kohlenstoffdioxid zu GCMA in Gegenwart eines oder mehrerer (zusätzlicher) Polymerisationsstabilisatoren erfolgt.

Als Polymerisationsstabilisatoren eigenen sich die zuvor genannten Polymerisationsstabilisatoren, wobei MeHQ und/oder Phenothiazin bevorzugt sind.

Die Gesamtmenge an Polymerisationsstabilisator, die in dem erfindungsgemäßen Verfahren eingesetzt wird, beträgt bevorzugt 0,005 bis 0,15 und weiter bevorzugt 0,05 bis 0,15 Gewichtsprozent, bezogen auf die Gesamtmenge des eingesetzten GMA, wobei die im GMA enthaltene Menge an Stabilisator unberücksichtigt bleibt.

Ist das eingesetzte GMA weitgehend zu GCMA umgesetzt wird die erhaltene Reaktionsmischung aufgearbeitet um GCMA aus der Reaktionsmischung zu isolieren. "Weitgehend" bedeutet in diesem Zusammenhang, dass 90 bis 100 Gewichtsprozent, bevorzugt 95 bis 100 Gewichtsprozent, besonders bevorzugt 98 bis 100 Gewichtsprozent des eingesetzten GMA zu GCMA umgesetzt sind.

Die Aufarbeitung der Reaktionsmischung umfasst einen oder mehrere Extraktionsschritte und/oder einen oder mehrere Destillationsschritte. Um den Katalysator zumindest weitgehend aus der Reaktionsmischung abzutrennen kann es von Vorteil sein, dass die Aufarbeitung zusätzlich einen oder mehrere Filtrations-, Zentrifugations-, Absorptions- und/oder Sedimentationsschritte umfasst. Um in der Reaktionsmischung noch enthaltenes GMA zumindest teilweise zu zersetzten kann es von Vorteil sein, dass die Aufarbeitung zusätzlich die Zugabe einer oder mehrerer Säuren, beispielsweise Ameisensäure, Essigsäure, Phosphorsäure und/oder Phosphinsäure umfasst, wobei Phosphorsäure und/oder Phosphinsäure bevorzugt sind. Die Zugabe einer oder mehrerer Säuren, hat den Vorteil, dass in der Reaktionsmischung enthaltenes GCMA nicht oder nur in geringem Maße zersetzt wird. Ein oder mehrere Destillationsschritte dienen der Abtrennung der leichter als GCMA flüchtigen Verbindungen.

Bei der Reihenfolge der oben genannten Verfahrensschritte zur Aufarbeitung der Reaktionsmischung lässt sich der Fachmann von praktischen Überlegungen leiten.

So kann die Reaktionsmischung beispielsweise filtriert, dem Filtrat eine oder mehrere Säuren zugegeben, die erhaltene Mischung in einem oder mehreren Schritten extrahiert und die erhaltene organische Phase in einem oder mehreren Schritten destilliert werden.

Eine oder mehrere Säuren können der Reaktionsmischung aber auch vor der Filtration oder nach der Extraktion zugegeben werden. Auch ist es möglich, zusätzlich dem nach der Destillation im Sumpf enthaltenen GCMA eine oder mehrere Säuren zuzugeben. Dies kann beispielsweise einen positiven Einfluss auf die Farbe des im Sumpf enthaltenen GCMAs haben.

Die Säure kann in konzentrierter Form oder in Form ihrer wässrigen Lösung bei der Aufarbeitung der Reaktionsmischung eingesetzt werden. Ameisensäure, Essigsäure, Phosphorsäure oder Phosphinsäure können in konzentrierter Form oder in Form ihrer wässrigen Lösungen, beispielsweise als 1 bis 99 prozentige wässrige Lösung bei der Aufarbeitung der Reaktionsmischung eingesetzt werden. Die Prozentangaben beziehen sich auf das Gesamtgewicht der zugegeben wässrigen Säurelösung. Es ist bevorzugt Phosphorsäure und/oder Phosphinsäure in Form ihrer wässrigen Lösungen der Reaktionsmischung zuzugeben.

Die Gesamtmenge an Säure die bei der Aufarbeitung eingesetzt wird, richtet sich nach der in der Reaktionsmischung noch enthaltenen Restmenge an GMA. Die in der Reaktionsmischung noch enthaltene Restmenge an GMA kann beispielsweise mittels GC -Techniken detektiert und quantifiziert werden. Zur sicheren Quantifizierung von GMA-Spuren <0,2% können auch HPLC- bzw. UPLC-Techniken verwendet werden. Es ist bevorzugt, die Gesamtmenge der eingesetzten Säure, bzw. Säuren derart zu bemessen, dass diese in einem stöchiometrischen Überschuss von 5 bis 1000 Prozent bezogen auf die in der Reaktionsmischung enthaltene Restmenge an GMA der Reaktionsmischung zugegeben werden.

Es ist bevorzugt, eine oder mehrere Säuren bei einer Temperatur von 10°C bis 100°C der Reaktionsmischung zuzugeben. Es ist besonders bevorzugt, eine oder mehrere Säuren bei einer Temperatur von 10°C bis 60°C und insbesondere bei einer Temperatur von 30°C bis 50°C der Reaktionsmischung zuzugeben.

Es ist besonders bevorzugt Phosphorsäure und/oder Phosphinsäure bei einer Temperatur von 10°C bis 100°C der Reaktionsmischung zuzugeben und insbesondere bei einer Temperatur von 30°C bis 50°C.

Verfahrenstechnisch können im erfindungsgemäßen Verfahren alle an sich bekannten Filtrationsverfahren und -apparate eingesetzt werden, z.B. solche, die in Ullmann's Encyclopedia of Industrial Chemistry, 7th ed, 2013 Electronic Release, Kapitel: Filtration, 1. Fundamentals und Filtration 2. Equipment, beschrieben sind. Beispielsweise können dies Kerzenfilter, Filterpressen, Tellerdruckfilter, Beutelfilter oder Trommelfilter sein. Vorzugsweise werden Kerzenfilter oder Tellerdruckfilter eingesetzt. Die Filtration kann mit oder ohne Filterhilfsmittel durchgeführt werden. Geeignete Filterhilfsmittel sind Filterhilfsmittel basierend auf Kieselgur, Perlit und Cellulose.

Verfahrenstechnisch können im erfindungsgemäßen Verfahren alle an sich bekannten Zentrifugationsverfahren und -apparate bzw. Sedimentationsverfahren und -apparate eingesetzt werden, z.B. solche, die in Ullmann's Encyclopedia of Industrial Chemistry, 7th ed, 2013 Electronic Release, Kapitel: Centrifuges, Filtering und Centrifuges, Sedimenting, beschrieben sind.

In einem Extraktionsschritt wird die Reaktionsmischung mit Wasser versetzt und die Phasen anschließend getrennt. Das Mengenverhältnis Wasser zu Reaktionsmischung, das zu Extraktionszwecken verwendet wird kann über einen weiten Bereich variieren. Vorteilhafte Mengenverhältnisse können vom Fachmann durch wenige Routineversuche bestimmt werden, oder erschließen sich ihm aus seinem allgemeinen Fachwissen oder anhand von praktischen Überlegungen.

Das Wasser kann zusätzlich ein gelöstes Salz enthalten. Bei dem Salz handelt es sich beispielsweise um Natriumchlorid, Kaliumchlorid, Ammoniumchlorid, Ammoniumsulfat, oder um beliebige Mischungen dieser. Es ist bevorzugt, dass es sich bei dem Salz um Natriumchlorid handelt. Die Menge des Salzes entspricht den üblichen Mengen, die zu Extraktionszwecken verwendet werden. Vorteilhafte Mengen können vom Fachmann durch wenige Routineversuche bestimmt werden, oder erschließen sich ihm aus seinem allgemeinen Fachwissen oder anhand von praktischen Überlegungen.

Die Zugabe des Wassers oder der wässrigen Lösung eines Salzes erfolgt bei einer Temperatur von 10 bis 70°C, bevorzugt bei einer Temperatur von 40 bis 60°C.

Zusätzlich kann ein organisches Lösungsmittel, das schlecht in Wasser löslich ist, der Reaktionsmischung zugesetzt werden. Dies kann beispielsweise dazu dienen, die Phasentrennung zu erleichtern. Ein organisches Lösungsmittel, das schlecht in Wasser löslich ist, hat eine Löslichkeit in Wasser von weniger als 10g/l Wasser bei 20°C, bevorzugt weniger als 1g/l Wasser bei 20°C.

Das Mengenverhältnis Reaktionsmischung : wässrige Lösung eines Salzes kann über weite Bereiche variieren. Vorteilhafte Mengenverhältnisse können vom Fachmann durch wenige Routineversuche bestimmt werden, oder erschließen sich ihm aus seinem allgemeinen Fachwissen oder anhand von praktischen Überlegungen.

Verfahrenstechnisch können für eine Extraktion in den erfindungsgemäßen Verfahren alle an sich bekannten Extraktions- und Waschverfahren und -apparate eingesetzt werden, beispielsweise solche, die in Ullmann's Encyclopedia of Industrial Chemistry, 6th Ed., 1999 Electronic Release, Kapitel "Liquid - Liquid Extraction - Apparatus" beschrieben sind. Beispielsweise können dies ein- oder mehrstufige, bevorzugt einstufige Extraktionen sowie Extraktionen in Gleich- oder Gegenstromfahrweise sein. Behälter die sich für die Extraktion eignen sind beispielsweise Rührbehälter, Kolonnen oder Mixer-Settler-Apparaturen.

In einem Destillationsschritt werden die leichter als GCMA flüchtigen Verbindungen aus der Reaktionsmischung zumindest weitgehend destillativ abgetrennt. GCMA bleibt als Sumpffraktion zurück.

Zur destillativen Abtrennung der leichter flüchtigen Verbindungen eignen sich in der Regel alle Apparaturen zur destillativen Auftrennung von Reaktionsgemischen, die flüssige Komponenten enthalten. Geeignete Apparaturen umfassen Destillationskolonnen, wie Bodenkolonnen, die mit Glockenböden, Siebplatten, Siebböden, Packungen oder Füllkörpern ausgerüstet sein können, oder Drehbandkolonnen-Verdampfer, wie Dünnschichtverdampfer, Fallfilmverdampfer, Zwangsumlaufverdampfer, Sambay-Verdampfer, etc. und Kombinationen davon. Es können ein oder mehrere Destillationsschritte hintereinandergeschaltet sein. Die Destillationsschritte können in gleichen oder in verschiedenen Apparaturen erfolgen.

Bei der Auswahl geeigneter Temperatur- und Druckbereiche zur destillativen Abtrennung der leichter siedenden Verbindungen lässt sich der Fachmann von den physikalischen Gegebenheiten der Trennaufgabe (beispielsweise Dampfdruckkurven) sowie von seinem allgemeinen Fachwissen und von praktischen Überlegungen leiten.

Vorteilhafterweise muss das nach dem erfindungsgemäßen Verfahren hergestellte GCMA selbst keiner destillativen oder rektifikativen Aufreinigung unterzogen werden, um GCMA in hoher Reinheit zu isolieren. Folglich kann die Isolierung von GCMA in relativ simplen Apparaturen erfolgen. Auch wird die thermische Belastung von GCMA bei der Isolierung reduziert, wodurch die Bildung von Nebenprodukten minimiert werden kann. Eine hohe Reinheit bedeutet in diesem Zusammenhang, dass der Anteil an Nebenprodukten im GCMA 0 bis 5 Gewichtsprozent, bevorzugt 0 bis 3 Gewichtsprozent und besonders bevorzugt 0 bis 2 Gewichtsprozent beträgt, bezogen auf die Gesamtmenge des isolierten GCMAs.

Um die Farbzahl des nach den erfindungsgemäßen Verfahren hergestellten GCMAs weiter zu reduzieren kann es von Vorteil sein, dass GCMA destilliert oder rektifiziert wird. Hierzu kann GCMA beispielsweise direkt durch Destillation oder Rektifikation aus der Reaktionsmischung isoliert werden. Wurde GCMA durch Extraktion und/oder destillative Abtrennung der leichter siedenden Verbindungen isoliert, kann auch das so erhaltene GCMA einer Destillation oder Rektifikation unterzogen werden. Generell wird durch das erfindungsgemäße Verfahren jedoch GCMA mit ausreichend geringer Farbzahl erhalten, wodurch eine Destillation oder Rektifikation des GCMAs selbst nicht notwendig ist.

Das erfindungsgemäße Verfahren bietet den Vorteil, dass GCMA in hoher Selektivität bei niedrigen Drücken und unter Verwendung eines einfach zugänglichen Katalysators hergestellt werden kann. Das erfindungsgemäße Verfahren stellt damit ein wirtschaftlich attraktives Verfahren zur Herstellung von GCMA dar.

Ein weiterer Vorteil des erfindungsgemäßen Verfahrens liegt beispielsweise darin, dass GCMA in hoher Qualität hergestellt werden kann. Eine hohe Qualität des nach dem erfindungsgemäßen Verfahren hergestellten GCMAs zeigt sich beispielsweise in einer hohen Reinheit und/oder einer geringen Farbzahl.

Durch das erfindungsgemäße Verfahren hergestelltes GCMA eignet sich beispielsweise zur Herstellung von Copolymeren. Neben Glycerincarbonatmethacrylat enthalten diese Copolymere bevorzugt auch Acrylate, Methacrylate und/oder Vinylmonomere in einpolymerisierter Form.

Über die Reaktivität der Carbonatgruppe des einpolymerisierten GCMAs ist es möglich, GCMA enthaltende Copolymere zu modifizieren und damit in ihren Eigenschaften gezielt zu verändern.

So werden Copolymere, die GCMA als Comonomer in einpolymerisierter Form enthalten, beispielsweise als Vernetzer eingesetzt. Die Carbonatgruppe des einpolymerisierten GCMAs ermöglicht beispielsweise Vernetzungsreaktionen mit Polymeren, die freie Aminogruppen, Hydrazidgruppen, Hydrazongruppen, Carbonsäuregruppen, Anhydridgruppen und/oder Hydroxygruppen aufweisen.

Experimentalteil:
Die Reinheit wurde mittels Gaschromatographie bestimmt. Als Lösemittel für die Proben wurde Dichlormethan der Fa. Aldrich verwendet, Reinheit 99,8%.

Als Gerät wurde ein Gaschromatograph der Firma Agilent (6890N) mit FID-Detektor & Säule RTX-5-Amin * 15 m *0,25 mm * 0,25 $\mu$m der Firma Restek benutzt.

Als Temperaturprogramm wurde eingestellt: 60°C Start, dann mit 15°C/min auf 300°C, 10 min bei 300°C, Gesamtlaufzeit 26 min. Die Prozent-Angaben zur Reinheit und Zusammensetzung der Reaktionslösungen wurden mittels Gaschromatographie als Flächenprozent ermittelt und nicht weiter quantifiziert. Sie werden im Folgenden als Fl.% oder Flächen% angegeben.

Die Prozentangaben zum Umsatz wurden nach der folgenden Formel berechnet:

$$Umsatz(\%) = \frac{GCMA(Fl.\%) \times 100}{GCMA(Fl.\%) + GMA(Fl.\%)}$$

Der GMA Gehalt im ppm-Bereich wurde mittels UPLC und externem Standard quantifiziert.

Ppm-Angaben beziehen sich auf mg/Kg. Als Lösemittel für die Proben wurde Acetonitril / Wasser im Volumenverhältnis 1:1 verwendet. Als Gerät wurde ein UPLC der Firma Waters mit UV-Detektor & Säule ACQUITY UPLC BEH C18 1.7$\mu$m 2.1x150mm der Firma Waters benutzt. Als Eluenten dienten Acetonitril und Wasser in Gradientenfahrweise (0,5 mL/min). Gesamtlaufzeit 14 min, Equilibrierzeit 4 min, Säulentemperatur 45°C sowie Anfangsdruck ca. 11000 psi.

Die Selektivität wurde nach der folgenden Formel bestimmt:

$$Selektivit\ddot{a}t(\%) = \frac{GCMA(Fl.\%) \times 100}{GCMA(Fl.\%) + \sum Nebenprodukte(Fl.\%)}$$

Die Hazen Farbzahl sowie die Jodfarbzahl wurden mit einem Farbzahlmessgerät der Fa. Hach Lange (LICO 620) gemessen und für Normlichtart C und 2°-Normalbeobachter entsprechend DIN 5033 berechnet.
Verwendete Edukte:

|  | Quelle | Reinheit | Stabilisierung |
|---|---|---|---|
| GMA | Novasol S.A. | >98 | 100 +/- 20 ppm MeHQ |
| Kohlenstoffdioxid | Praxair | 99,9% | |
| Kaliumiodid | Honeywell Sigma-Aldrich | >99,5% (p.a.) | |
| Acetonitril | Honywell | >99,5% (GC) | |
| Phosphorsäure | Sigma-Aldrich | 85 % in $H_2O$ | |
| Phosphinsäure | Sigma-Aldrich | 50% in H2O | |

Erfindungsgemäße Beispiele:

Beispiel 1

7mg PTZ und 4,15 g Acetonitril werden in einem Rundkolben mit Intensivkühler, Magnetrührer, Thermometer, $CO_2$-Einleitung sowie Druckausgleich zur Umgebung vorgelegt. 41,5 g GMA sowie 4,75 g KI werden hinzugegeben. $CO_2$ (ca. 2 L/h) sowie 0,5L/h Luft werden in die Reaktionsmischung eingeleitet. Die Reaktionsmischung wird auf eine Innentemperatur von 80 bis 85°C erwärmt. In regelmäßigen Abständen werden Proben gezogen und gaschromatographisch ausgewertet. Nach 6h beträgt der Umsatz von GMA zu GCMA 80,25%. Die Mischung enthält (das Lösemittel Acetonitril herausgerechnet und nicht GC-gängige Komponenten unberücksichtigt) 79,38 Flächen% GCMA und 19,53 Flächen% GMA. Die Summe der Nebenprodukte beträgt 1,09 Flächen%. Die Selektivität beträgt 98,6%. Die Reaktionslösung ist farblos.

Beispiel 2

7mg PTZ und 0,42 g n-Butanol werden in einem Rundkolben mit Intensivkühler, Magnetrührer, Thermometer, $CO_2$-Einleitung sowie Druckausgleich zur Umgebung vorgelegt. 41,5 g GMA sowie 4,75 g KI werden hinzugegeben. $CO_2$ (ca. 2 L/h) sowie 0,5L/h Luft werden in die Reaktionsmischung eingeleitet. Die Reaktionsmischung wird auf eine Innentemperatur von 80 bis 85°C erwärmt. In regelmäßigen Abständen werden Proben gezogen und gaschromatographisch ausgewertet. Nach 6h beträgt der Umsatz von GMA zu GCMA 82,7%. Die Mischung enthält (nicht GC-gängige Komponenten unberücksichtigt) 81,63 Flächen% GCMA und 17,04 Flächen% GMA. Die Summe der Nebenprodukte beträgt 0,9 Flächen%. Die Menge an n-Butanol beträgt 0,43 Flächen%. Die Selektivität beträgt 98,9%. Die Reaktionslösung ist farblos.

Beispiel 3

7mg PTZ und 2,08 g sek-Butanol werden in einem Rundkolben mit Intensivkühler, Magnetrührer, Thermometer, $CO_2$-Einleitung sowie Druckausgleich zur Umgebung vorgelegt. 41,5 g GMA sowie 4,75 g KI werden hinzugegeben. $CO_2$ (ca. 2 L/h) sowie 0,5L/h Luft werden in die Reaktionsmischung eingeleitet. Die Reaktionsmischung wird auf eine Innentemperatur von 80 bis 85° C erwärmt. In regelmäßigen Abständen werden Proben gezogen und gaschromatographisch ausgewertet. Nach 6h beträgt der Umsatz von GMA zu GCMA 84,3%. Die Mischung enthält (nicht GC-gängige Komponenten unberücksichtigt) 80,59 Flächen% GCMA und 15,04 Flächen% GMA. Die Summe der Nebenprodukte beträgt 1,72 Flächen%. Die Menge an sek-Butanol beträgt 2,65 Flächen%. Die Selektivität beträgt 97,9%. Die Reaktionslösung ist farblos.

Beispiel 4

7mg PTZ und 2,08 g Acetonitril werden in einem Rundkolben mit Intensivkühler, Magnetrührer, Thermometer,

CO$_2$-Einleitung sowie Druckausgleich vorgelegt. 41,5 g GMA sowie 4,75 g KI werden hinzugegeben. CO$_2$ (ca. 2 L/h) sowie 0,5L/h Luft werden in die Reaktionsmischung eingeleitet. Die Reaktionsmischung wird auf eine Innentemperatur von 80 bis 85°C erwärmt. In regelmäßigen Abständen werden Proben gezogen und gaschromatographisch ausgewertet. Nach 6h beträgt der Umsatz von GMA zu GCMA 80,9%. Die Mischung enthält (das Lösemittel Acetonitril herausgerechnet und nicht GC-gängige Komponenten unberücksichtigt) 80,03 Flächen% GCMA und 18,85 Flächen% GMA. Die Summe der Nebenprodukte beträgt 1,12 Flächen%. Die Selektivität beträgt 98,6%. Die Reaktionslösung ist farblos.

Beispiel 5

7mg PTZ und 2,08 g Isopropanol werden in einem Rundkolben mit Intensivkühler, Magnetrührer, Thermometer, CO$_2$-Einleitung sowie Druckausgleich vorgelegt. 41,5 g GMA sowie 2,37 g KI werden hinzugegeben. CO$_2$ (ca. 2 L/h) sowie 0,5L/h Luft werden in die Reaktionsmischung eingeleitet. Die Reaktionsmischung wird auf eine Innentemperatur von 80 bis 85°C erwärmt. In regelmäßigen Abständen werden Proben gezogen und gaschromatographisch ausgewertet. Nach 8h beträgt der Umsatz von GMA zu GCMA 98,1%. Die Mischung enthält (das Lösemittel Isopropanol herausgerechnet und nicht GC-gängige Komponenten unberücksichtigt) 96,68 Flächen% GCMA und 1,91 Flächen% GMA. Die Summe der Nebenprodukte beträgt 1,41 Flächen%. Die Selektivität beträgt 98,1%. Die Reaktionslösung ist leicht gelblich.

Beispiel 6

7mg PTZ und 2,08 g Acetonitril werden in einem Rundkolben mit Intensivkühler, Magnetrührer, Thermometer, CO$_2$-Einleitung sowie Druckausgleichvorgelegt. 41,5 g GMA sowie 4,75 g fein gemörsertes KI werden hinzugegeben. CO$_2$ (ca. 2 L/h) sowie 0,5L/h Luft werden in die Reaktionsmischung eingeleitet. Die Reaktionsmischung wird auf eine Innentemperatur von 80 bis 85° C erwärmt. In regelmäßigen Abständen werden Proben gezogen und gaschromatographisch ausgewertet. Nach 6h beträgt der Umsatz von GMA zu GCMA 80,3%. Die Mischung enthält (das Lösemittel Acetonitril herausgerechnet und nicht GC-gängige Komponenten unberücksichtigt) 79,5 Flächen% GCMA und 19,56 Flächen% GMA. Die Summe der Nebenprodukte beträgt 0,94 Flächen%. Die Selektivität beträgt 98,8%. Die Reaktionslösung ist farblos.

Beispiel 7

7mg PTZ und 0,42 g n-Butanol werden in einem Rundkolben mit Intensivkühler, Magnetrührer, Thermometer, CO$_2$-Einleitung sowie Druckausgleich vorgelegt. 41,5 g GMA sowie 4,75 g KI werden hinzugegeben. CO$_2$ (ca. 2 L/h) sowie 0,5L/h Luft werden in die Reaktionsmischung eingeleitet. Die Reaktionsmischung wird auf eine Innentemperatur von 80 bis 85°C erwärmt. In regelmäßigen Abständen werden Proben gezogen und gaschromatographisch ausgewertet. Nach 6h beträgt der Umsatz von GMA zu GCMA 81,5%. Die Mischung enthält (nicht GC-gängige Komponenten unberücksichtigt) 80,45 Flächen% GCMA und 18,24 Flächen% GMA. Die Summe der Nebenprodukte beträgt 0,99 Flächen%. Die Menge an n-Butanol beträgt 0,32 Flächen%. Die Selektivität beträgt 98,8%. Die Reaktionslösung ist farblos.

Beispiel 8

540 mg MeHQ, 11mg PTZ, 72,9g KI und 238g Acetonitril werden in einem Rundkolben mit Intensivkühler, Rührer, Thermometer, CO$_2$-Einleitung sowie Druckausgleich vorgelegt. 425g GMA werden hinzugegeben. CO$_2$ (ca. 5 L/h) sowie 1L/h Luft werden in die Reaktionsmischung eingeleitet. Die Reaktionsmischung wird auf eine Innentemperatur von 80 bis 85°C erwärmt. In regelmäßigen Abständen werden Proben gezogen und gaschromatographisch ausgewertet. Nach 27h beträgt der Umsatz von GMA zu GCMA 99,4%. Die Reaktionslösung enthält noch 0,56 Flächen% GMA. Die Reaktionsmischung wird nach Abkühlen im Vakuum konzentriert und zweimal mit 50 ml Wasser extrahiert und die Phasen getrennt. Nach Trocknen der organischen Phase über Natriumsulfat wird filtriert und das Filtrat nochmals im Vakuum konzentriert. Es werden 530g (98,2% Ausbeute) einer hellen Flüssigkeit in 97,2 Flächen% Reinheit erhalten. Der Restgehalt an GMA beträgt 1,6 Flächen%. Die Selektivität beträgt 98,8%. Die Hazen-Farbzahl beträgt 88.

Beispiel 9

1,14g MeHQ, 23mg PTZ, 103g KI und 504g Acetonitril werden in einem Rundkolben mit Intensivkühler, Rührer, Thermometer, CO$_2$-Einleitung sowie Druckausgleich vorgelegt. 900g GMA werden hinzugegeben. CO$_2$ (ca. 30 L/h) sowie 1L/h Luft werden in die Reaktionsmischung eingeleitet. Die Reaktionsmischung wird auf eine Innentemperatur von 80 bis 85°C erwärmt. In regelmäßigen Abständen werden Proben gezogen und gaschromatographisch ausgewertet. Nach 7 h wird die Einleitung auf ca. 10 L/h reduziert. Nach 15h beträgt der Umsatz von GMA zu GCMA 99,11%. Die Reaktionslösung enthält noch 0,64 Flächen% GMA.

Beispiel 10

2g einer analog zu Beispiel 9 hergestellten Reaktionsmischung, die einen Umsatz von GMA zu GCMA von 99,41% aufweist und noch 0,42 Flächen% GMA enthält (0,5 Flächen% ohne Integration des Lösemittel Acetonitril), werden mit 0,2 g Phosphinsäure (50 Gew.%) versetzt und 1,5h bei Raumtemperatur gerührt. Eine Probe wird gezogen und gaschomatographisch analysiert. Es sind noch 0,07 Flächen% GMA vorhanden (ohne Integration des Lösemittels).

Beispiel 11

2g einer analog zu Beispiel 9 hergestellten Reaktionsmischung, die einen Umsatz von GMA zu GCMA von 99,41% aufweist und noch 0,42 Flächen% GMA enthält (0,5 Flächen% ohne Integration des Lösemittel Acetonitril), werden mit 0,2 g Phosphorsäure (85 Gew. %) versetzt und 1,5h bei Raumtemperatur gerührt. Es sind noch 0,11 Flächen% GMA vorhanden (ohne Integration des Lösemittels).

Beispiel 12

2g einer analog zu Beispiel 9 hergestellten Reaktionsmischung, die einen Umsatz von GMA zu GCMA von 99,41% aufweist und noch 0,42 Flächen% GMA enthält (0,5 Flächen% ohne Integration des Lösemittel Acetonitril), werden mit 0,2 g Essigsäure (99%ig) versetzt und 1,5h bei Raumtemperatur gerührt. Es sind noch 0,2 Flächen% GMA vorhanden (ohne Integration des Lösemittels und der Essigsäure).

Beispiel 13

2g einer analog zu Beispiel 9 hergestellten Reaktionsmischung, die einen Umsatz von GMA zu GCMA von 99,41% aufweist und noch 0,42 Flächen% GMA enthält (0,5 Flächen% ohne Integration des Lösemittel Acetonitril), werden mit 0,2 g Ameisensäure (98%ig) versetzt und 1,5h bei Raumtemperatur gerührt. Es sind noch 0,14 Flächen% GMA vorhanden (ohne Integration des Lösemittels und der Ameisensäure).

Beispiel 14

Eine Reaktionsmischung analog Beispiel 9 wird nach Abkühlen mit 12g Phosphinsäure (50 Gew. %) versetzt und nach 30 min Rühren filtriert. Die Lösung wird im Vakuum konzentriert und zweimal mit 150 ml Wasser extrahiert sowie die Phasen getrennt. Anschließend wird mit wässriger $Na_2CO_3$-Lösung neutralisiert. Nach Phasentrennung wird die organische Phase nochmals im Vakuum konzentriert. Es werden 1153g einer hellen Flüssigkeit in 98,3 Flächen% Reinheit erhalten. Der Restgehalt an GMA beträgt <20ppm (UPLC). Die Hazen-Farbzahl beträgt 100.

Beispiel 15

2,8g MeHQ, 56mg PTZ, 252g KI und 1233g Acetonitril werden in einem beheizbaren Doppelwandreaktor mit Intensivkühler, Scheibenrührer, Thermometer, $CO_2$-Einleitung sowie Druckausgleich vorgelegt. 2200g GMA werden hinzugegeben. $CO_2$ (ca. 40 L/h) sowie 1L/h Luft werden in die Reaktionsmischung eingeleitet. Die Reaktionsmischung wird auf eine Innentemperatur von 85 bis 90°C erwärmt. In regelmäßigen Abständen werden Proben gezogen und gaschromatographisch ausgewertet. Nach 13h beträgt der Umsatz von GMA zu GCMA 99,46%. Die Reaktionslösung enthält noch 0,36 Flächen% GMA. Im Vakuum wird Acetonitril abdestilliert und anschließend auf 50°C abgekühlt. 500 mL Wasser werden hinzugegeben und extrahiert. Die wässrige Phase wird abgetrennt. Die Reaktionsmischung wird mit 36g Phosphinsäure (50 Gew. %) versetzt und noch zweimal bei Raumtemperatur mit 500 mL Wasser extrahiert. Die organische Phase wird mit wässriger $Na_2CO_3$-Lösung neutralisiert, die wässrige Phase abgetrennt und im Vakuum konzentriert. Es werden 2798g einer hellen Flüssigkeit in 98,7 Flächen% Reinheit erhalten. Der Restgehalt an GMA beträgt <20ppm (UPLC). Die Hazen-Farbzahl beträgt 32.

Beispiel 16

3,05g MeHQ, 348mg PTZ, 275g KI und 24g tert.Butanol werden in einem beheizbaren Doppelwandreaktor mit Intensivkühler, Scheibenrührer, Thermometer, $CO_2$-Einleitung sowie Druckausgleich vorgelegt. 2400g GMA werden hinzugegeben. $CO_2$ (ca. 35 L/h) sowie 1L/h Luft werden in die Reaktionsmischung eingeleitet. Die Reaktionsmischung wird auf eine Innentemperatur von 90 bis 95°C erwärmt. In regelmäßigen Abständen werden Proben gezogen und gaschromatographisch ausgewertet. Nach 13h beträgt der Umsatz von GMA zu GCMA 99,65%. Es wurden 871 g $CO_2$ verbraucht. Die Reaktionslösung enthält noch 0,34 Flächen% GMA. Nach Abkühlen auf 40°C werden 500 mL Wasser hinzugegeben und extrahiert. Die wässrige Phase wird abgetrennt. Die Reaktionsmischung wird mit 31g Phosphinsäure (50 Gew.%) versetzt und noch zweimal mit 500 mL Wasser extrahiert. Die organische Phase wird mit wässriger $Na_2CO_3$-Lösung neutralisiert, die wässrige Phase abgetrennt und im Vakuum konzentriert. Weitere 15 g Phosphinsäure (50 Gew. %) werden hinzugefügt. Es werden 3059 g einer hellen Flüssigkeit in 98 Flächen% Reinheit erhalten. Der Restgehalt an GMA beträgt <20ppm (UPLC). Die Hazen-Farbzahl beträgt 34.

Beispiel 17

4,3g MeHQ, 86mg PTZ, 387g KI und 169g Acetonitril werden in einem beheizbaren Doppelwandreaktor mit Intensivkühler, Scheibenrührer, Thermometer, $CO_2$-Einleitung sowie Druckausgleich vorgelegt. 3386g GMA werden hinzugegeben. $CO_2$ (ca. 40 L/h) sowie 1L/h Luft werden in die Reaktionsmischung eingeleitet. Die Reaktionsmischung wird auf eine Innentemperatur von 90 bis 95°C erhitzt. In regelmäßigen Abständen werden Proben gezogen und gaschromatographisch ausgewertet. Nach 16h beträgt der Umsatz von GMA zu GCMA 99,44%. Die Reaktionslösung enthält noch 0,53 Flächen% GMA. Nach Abkühlen auf 60°C werden 500 mL Wasser hinzugegeben und extrahiert. Die wässrige Phase wird abgetrennt. Die Reaktionsmischung wird mit 31g Phosphinsäure (50 Gew.%) versetzt und noch zweimal bei 40 bis 50°C mit 500 mL Wasser extrahiert. Die organische Phase wird mit wässriger $Na_2CO_3$-Lösung neutralisiert, die wässrige Phase abgetrennt und im Vakuum konzentriert. Es werden 4299g einer hellen Flüssigkeit in 98,3 Flächen% Reinheit erhalten. Der Restgehalt an GMA beträgt <20ppm (UPLC). Die Hazen-Farbzahl beträgt 193.

Beispiel 18

2,92g MeHQ, 58mg PTZ, 263g KI und 115g Acetonitril werden in einem beheizbaren Doppelwandreaktor mit Intensivkühler, Scheibenrührer, Thermometer, $CO_2$-Einleitung sowie Druckausgleich vorgelegt. 2300g GMA werden hinzugegeben. $CO_2$ (ca. 50 bis 70 L/h) wird in die Reaktionsmischung eingeleitet. Die Reaktionsmischung wird auf eine Innentemperatur von 95 bis 98° C erwärmt. In regelmäßigen Abständen werden Proben gezogen und gaschromatographisch ausgewertet. Nach 7h beträgt der Umsatz von GMA zu GCMA 99,04%. Es wurden 815 g $CO_2$ verbraucht. Die Reaktionsmischung wird nach Abkühlen mit 31g Phosphinsäure (50 Gew.%ig) versetzt und nach 30 min Rühren filtriert. Die Lösung wird bei 40°C zweimal mit 500 ml Wasser extrahiert sowie die Phasen getrennt. Anschließend wird mit wässriger $Na_2CO_3$-Lösung neutralisiert und nochmals mit 500 ml Wasser extrahiert. Nach Phasentrennung werden 9g Phosphinsäure (50 Gew.%) hinzugefügt. Nach 20 min wird erneut mit wässriger $Na_2CO_3$-Lösung neutralisiert, die Phasen getrennt und die organische Phase im Vakuum konzentriert. Es werden 2864g einer hellen Flüssigkeit erhalten, die nochmals filtriert werden. Die Reinheit beträgt 98,5 Flächen%. Der Restgehalt an GMA beträgt <20ppm (UPLC). Die Hazen-Farbzahl beträgt 20.

Vergleichsbeispiele:

Beispiel V1

7mg PTZ werden in einem Rundkolben mit Intensivkühler, Magnetrührer, Thermometer, $CO_2$-Einleitung sowie Druckausgleich vorgelegt. 41,5 g GMA sowie 4,75 g KI werden hinzugegeben. $CO_2$ (ca. 2 L/h) sowie 0,5L/h Luft werden in die Reaktionsmischung eingeleitet. Die Reaktionsmischung wird auf eine Innentemperatur von 80 bis 85°C Innentemperatur erwärmt. In regelmäßigen Abständen werden Proben gezogen und gaschromatographisch ausgewertet. Nach 6h beträgt der Umsatz von GMA zu GCMA 68,9%. Die Mischung enthält (nicht GC-gängige Komponenten unberücksichtigt) 68,41 Flächen% GCMA und 30,9 Flächen% GMA. Die Summe der Nebenprodukte beträgt 0,69 Flächen%. Die Selektivität beträgt 99%. Die Reaktionslösung ist farblos. Es ist erkennbar, dass die Umsetzung von GMA zu GCMA in Abwesenheit eines geeigneten Lösungsmittels bei Umgebungsdruck deutlich langsamer erfolgt.

Beispiel V2

7mg PTZ und 2,08 g Triethylamin werden in einem Rundkolben mit Intensivkühler, Magnetrührer, Thermometer, $CO_2$-Einleitung sowie Druckausgleich vorgelegt. 41,5 g GMA sowie 4,75 g KI werden hinzugegeben. $CO_2$ (ca. 2 L/h) sowie 0,5L/h Luft werden in die Reaktionsmischung eingeleitet. Die Reaktionsmischung wird auf eine Innentemperatur von 70 bis 74°C erwärmt. In regelmäßigen Abständen werden Proben gezogen und gaschromatographisch ausgewertet. Nach 7h beträgt der Umsatz von GMA zu GCMA 84,4%. Die Mischung enthält (nicht GC-gängige Komponenten unberücksichtigt) 79,21 Flächen% GCMA und 14,7 Flächen% GMA. Die Summe der Nebenprodukte beträgt 5,97 Flächen%. Die Selektivität beträgt 93%. Die Reaktionslösung ist orange.

Beispiel V3

17mg PTZ und 4,35 g Triethylamin werden in einem Rundkolben mit Intensivkühler, Magnetrührer, Thermometer, $CO_2$-Einleitung sowie Druckausgleich vorgelegt. 42 g GMA sowie 7,2 g KI werden hinzugegeben. $CO_2$ (ca. 2 L/h) wird in die Reaktionsmischung eingeleitet. Die Reaktionsmischung wird auf eine Innentemperatur von 71 bis 72°C erwärmt. In regelmäßigen Abständen werden Proben gezogen und gaschromatographisch ausgewertet. Nach 28h beträgt der Umsatz von GMA zu GCMA 99,26%. Die Mischung enthält (nicht GC-gängige Komponenten unberücksichtigt) 91,07 Flächen% GCMA und 0,68 Flächen% GMA. Die Summe der Nebenprodukte beträgt 8,14 Flächen%. Die Selektivität beträgt 91,8%.

Die Reaktionsmischung wird aufgearbeitet. Der Kolbeninhalt wird mit 10mL Ethylacetat versetzt, in einen Scheidetrichter überführt, 30 x mit je 30mL Wasser extrahiert. Die vereinten wässrigen Phasen werden mit 20mL Ethylacetat extrahiert. Die vereinten organischen Phasen werden noch einmal mit 20 mL ges. NaCl-Lösung extrahiert. Nach Phasentrennung wird die organische Phase über Natriumsulfat getrocknet, filtriert und mit Dichlormethan nachgewaschen. Das Filtrat wird im Vakuum konzentriert. Es werden 50,4 g einer braunen Lösung in 93,9% Reinheit erhalten. Die Ausbeute beträgt 94,5%. Die Hazenfarbzahl war nicht mehr messbar. Die Jod-Farbzahl beträgt 23.

**Patentansprüche**

1. Verfahren zur Herstellung von Glycerincarbonatmethacrylat, wobei Glycidylmethacrylat in Gegenwart eines Katalysators und eines Lösungsmittels mit Kohlenstoffdioxid umgesetzt wird,
   **dadurch gekennzeichnet, dass**
   es sich beim Katalysator um Kaliumiodid handelt, es sich bei dem Lösungsmittel um Acetonitril, ein oder mehrere Mono-Alkohole, oder eine beliebe Mischung aus Acetonitril und einem oder mehreren Mono-Alkoholen handelt und die Umsetzung von Glycidylmethacrylat mit Kohlenstoffdioxid bei einem Druck von 0,5 bis 5 bar erfolgt.

2. Verfahren nach Anspruch 1, wobei die Reaktionstemperatur 50 bis 100 °C beträgt.

3. Verfahren nach einem der Ansprüche 1 bis 2, wobei der Druck 0,8 bis 1,5 bar beträgt.

4. Verfahren nach einem der Ansprüche 1 bis 3, wobei es sich um ein diskontinuierliches Verfahren handelt.

5. Verfahren nach einem der Ansprüche 1 bis 4, wobei es sich bei dem Mono-Alkohol um Ethanol, n-Propanol, iso-Propanol, n-Butanol, sek-Butanol, iso-Butanol oder tert-Butanol handelt.

6. Verfahren nach einem der Ansprüche 1 bis 5, wobei die Gesamtmenge des eingesetzten Lösungsmittels 0,1 bis 50 Gewichtsprozent bezogen auf die Gesamtmenge des eingesetzten Glycidylmethacrylats beträgt.

7. Verfahren nach einem er Ansprüche 1 bis 6, wobei es sich bei dem Lösungsmittel um Acetonitril handelt und die Gesamtmenge des eingesetzten Lösungsmittel 2 bis 40 Gewichtsprozent bezogen auf die Gesamtmenge des eingesetzten Glycidylmethacrylats beträgt.

8. Verfahren nach einem der Ansprüche 1 bis 7, wobei das Molverhältnis zwischen der Gesamtmenge des eingesetzten Katalysators und der Gesamtmenge des eingesetzten Glycidylmethacrylats 0,005 : 1 bis 0,5 : 1 beträgt.

9. Verfahren nach einem der Ansprüche 1 bis 8, wobei Kohlenstoffdioxid als kohlenstoffdioxidhaltiges Gas in die Reaktionsmischung, enthaltend den Katalysator, das Lösungsmittel, noch nicht umgesetztes GMA und optional bereits gebildetes GCMA und/oder in den Gasraum oberhalb der Reaktionsmischung eingeleitet wird.

10. Verfahren nach Anspruch 9, wobei das kohlenstoffdioxidhaltige Gas kontinuierlich eingeleitet wird.

11. Verfahren nach Anspruch 1 bis 10, wobei ein sauerstoffhaltiges Gas in die Reaktionsmischung, enthaltend den Katalysator, das Lösungsmittel, noch nicht umgesetztes GMA und optional bereits gebildetes GCMA und/oder in den Gasraum oberhalb der Reaktionsmischung eingeleitet wird.

12. Verfahren nach einem der Ansprüche 1 bis 11, wobei die Umsetzung von Glycidylmethacrylat zu Glycerincarbonatmethacrylat in Gegenwart eines Stabilisators stattfindet und es sich bei dem Stabilisator um Phenothiazin, ein oder mehrere phenolische Verbindungen, ein oder mehrere N-Oxyle oder eine beliebige Mischung der zuvor genannten Stabilisatoren handelt.

**13.** Verfahren nach einem der Ansprüche 1 bis 12, wobei die nach weitgehender Umsetzung des eingesetzten Glycidylmethacrylats zu Glycerincarbonatmethacrylat erhaltene Reaktionsmischung aufgearbeitet wird und die Aufarbeitung einen oder mehrere Extraktionsschritte und/oder einen oder mehrere Destillationsschritte umfasst.

**14.** Verfahren nach Anspruch 13, wobei die Aufarbeitung zusätzlich zu einem oder mehreren Extraktionsschritten und/oder Destillationsschritten die Zugabe einer oder mehrerer Säuren umfasst.

**15.** Verfahren nach Anspruch 14, wobei es sich bei der Säure um Phosphinsäure und/oder Phosphorsäure handelt.


**Claims**

**1.** A process for preparing glycerol carbonate methacrylate, wherein glycidyl methacrylate is reacted with carbon dioxide in the presence of a catalyst and a solvent,
wherein
the catalyst is potassium iodide, the solvent is acetonitrile, one or more monoalcohols, or any desired mixture of acetonitrile and one or more monoalcohols, and the reaction of glycidyl methacrylate with carbon dioxide is carried out at a pressure from 0.5 to 5 bar.

**2.** The process according to claim 1, wherein the reaction temperature is 50 to 100°C.

**3.** The process according to either of claims 1 or 2, wherein the pressure is 0.8 to 1.5 bar.

**4.** The process according to any of claims 1 to 3, wherein it is a batchwise process.

**5.** The process according to any of claims 1 to 4, wherein the monoalcohol is ethanol, n-propanol, isopropanol, n-butanol, sec-butanol, isobutanol or tert-butanol.

**6.** The process according to any of claims 1 to 5, wherein the total amount of solvent used is 0.1 to 50 percent by weight based on the total amount of glycidyl methacrylate used.

**7.** The process according to any of claims 1 to 6, wherein the solvent is acetonitrile and the total amount of solvent used is 2 to 40 percent by weight based on the total amount of glycidyl methacrylate used.

**8.** The process according to any of claims 1 to 7, wherein the molar ratio between the total amount of catalyst used and the total amount of glycidyl methacrylate used is 0.005:1 to 0.5:1.

**9.** The process according to any of claims 1 to 8, wherein carbon dioxide as the carbon dioxide-containing gas is fed into the reaction mixture comprising the catalyst, the solvent, GMA that has not yet reacted, and optionally any GCMA already formed, and/or into the gas space above the reaction mixture.

**10.** The process according to claim 9, wherein the carbon dioxide-containing gas is fed in continuously.

**11.** The process according to claim 1 to 10, wherein an oxygen-containing gas is fed into the reaction mixture comprising the catalyst, the solvent, GMA that has not yet reacted, and optionally any GCMA already formed, and/or into the gas space above the reaction mixture.

**12.** The process according to any of claims 1 to 11, wherein the reaction of glycidyl methacrylate to glycerol carbonate methacrylate takes place in the presence of a stabilizer and the stabilizer is phenothiazine, one or more phenolic compounds, one or more N-oxyls or any desired mixture of the abovementioned stabilizers.

**13.** The process according to any of claims 1 to 12, wherein the reaction mixture resulting from conversion of most of the glycidyl methacrylate used into glycerol carbonate methacrylate is worked up and the workup comprises one or more extraction steps and/or one or more distillation steps.

**14.** The process according to claim 13, wherein the workup includes, in addition to one or more extraction steps and/or

**EP 3 724 174 B1**

distillation steps, the addition of one or more acids.

15. The process according to claim 14, wherein the acid is phosphinic acid and/or phosphoric acid.

**Revendications**

1. Procédé pour la préparation de méthacrylate de carbonate de glycérol, dans lequel on fait réagir avec du dioxyde de carbone du méthacrylate de glycidyle en présence d'un catalyseur et d'un solvant,
**caractérisé en ce que**
pour ce qui est du catalyseur il s'agit d'iodure de potassium, pour ce qui est du solvant il s'agit d'acétonitrile, d'un ou de plusieurs monoalcools, ou d'un mélange quelconque d'acétonitrile et d'un ou de plusieurs monoalcools et la réaction du méthacrylate de glycidyle avec le dioxyde de carbone s'effectue sous une pression de 0,5 à 5 bars.

2. Procédé selon la revendication 1, dans lequel la température de réaction est de 50 à 100 °C.

3. Procédé selon l'une quelconque des revendications 1 et 2, dans lequel la pression est de 0,8 à 1,5 bars.

4. Procédé selon l'une quelconque des revendications 1 à 3, dans lequel il s'agit d'un procédé discontinu.

5. Procédé selon l'une quelconque des revendications 1 à 4, dans lequel pour ce qui est du monoalcool il s'agit de l'éthanol, du n-propanol, de l'isopropanol, du n-butanol, du sec-butanol, de l'isobutanol ou du tert-butanol.

6. Procédé selon l'une quelconque des revendications 1 à 5, dans lequel la quantité totale du solvant utilisé vaut de 0,1 à 50 pour cent en poids par rapport à la quantité totale du méthacrylate de glycidyle utilisé.

7. Procédé selon l'une quelconque des revendications 1 à 6, dans lequel pour ce qui est du solvant il s'agit d'acétonitrile et la quantité totale du solvant utilisé vaut de 2 à 40 pour cent en poids par rapport à la quantité totale du méthacrylate de glycidyle utilisé.

8. Procédé selon l'une quelconque des revendications 1 à 7, dans lequel le rapport molaire entre la quantité totale du catalyseur utilisé et la quantité totale du méthacrylate de glycidyle utilisé vaut de 0,005 : 1 à 0,5 : 1.

9. Procédé selon l'une quelconque des revendications 1 à 8, dans lequel le dioxyde de carbone est introduit sous forme de gaz contenant du dioxyde de carbone dans le mélange réactionnel contenant le catalyseur, le solvant, du GMA n'ayant pas encore réagi et en option du GCMA déjà formé et/ou dans l'atmosphère au-dessus du mélange réactionnel.

10. Procédé selon la revendication 9, dans lequel le gaz contenant du dioxyde de carbone est introduit en continu.

11. Procédé selon l'une quelconque des revendications 1 à 10, dans lequel un gaz contenant de l'oxygène est introduit dans le mélange réactionnel contenant le catalyseur, le solvant, du GMA n'ayant pas encore réagi et en option du GCMA déjà formé et/ou dans l'atmosphère au-dessus du mélange réactionnel.

12. Procédé selon l'une quelconque des revendications 1 à 11, dans lequel la conversion du méthacrylate de glycidyle en méthacrylate de carbonate de glycérol a lieu en présence d'un stabilisant et pour ce qui est du stabilisant il s'agit de phénolthiazine, d'un ou de plusieurs composés phénoliques, d'un ou de plusieurs N-oxyles ou d'un mélange quelconque des stabilisants précités.

13. Procédé selon l'une quelconque des revendications 1 à 12, dans lequel on soumet à un traitement final le mélange réactionnel obtenu après conversion en grande partie du méthacrylate de glycidyle utilisé en méthacrylate de carbonate de glycérol et le traitement final comprend une ou plusieurs étapes d'extraction et/ou une ou plusieurs étapes de distillation.

14. Procédé selon la revendication 13, dans lequel en plus d'une ou de plusieurs étapes d'extraction et/ou étapes de distillation le traitement final comprend l'addition d'un ou de plusieurs acides.

15. Procédé selon la revendication 14, dans lequel pour ce qui est de l'acide il s'agit d'acide phosphinique et/ou d'acide

phosphorique.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- Ullmann's Encyclopedia of Industrial Chemistry. 2013
- Liquid - Liquid Extraction - Apparatus. Ullmann's Encyclopedia of Industrial Chemistry. 1999